# EUROPÄISCHE PATENTANMELDUNG

(11) **EP 1 929 966 A2**
(43) Veröffentlichungstag der Anmeldung: **11.06.2008**
(21) Anmeldenummer: 07022913.3
(22) Anmeldetag: 27.11.2007
(51) Int. Cl.: A61B 17/50, A61B 17/22

(54) **Medizinisches Instrument zum Entfernen von Gegenständen aus engen Kanälen**

(30) Priorität: 08.12.2006 DE 102006057900
(71) Anmelder: University of Dundee, Dundee Dundee City DD1 4HN (GB)
(72) Erfinder: Brown, Stuart I., Dr., Fife KY16 8QX (GB); Frank, Timothy G., Dr., Fife KY16 9TY (GB); Kelly, Leslie, Fife KY15 5YL (GB); Mountain, Rodney, Fife KY15 4NB (GB); Rutherford, Ian, Dundee DD4 0RL (GB)
(74) Vertreter: Hofmeister, Frank

(57) **Zusammenfassung**

Die Erfindung betrifft ein medizinisches Instrument (1) zum Entfernen von Gegenständen (7) aus engen Kanälen (6), mit einem Schaft (2) und einem im Schaft (2) gelagerten Betätigungselement (4), an dessen distalem Ende eine hakenförmig Abwinklung (5) ausbildbar ist, wobei das distale Ende des Betätigungselements (4) über eine Handhabe (3) zwischen einer im Wesentlichen gestreckten Form und einer die hakenförmige Abwinklung (5) aufweisenden Form verstellbar ist. Um ein medizinisches Instrument (1) zu schaffen, das bei einfacher und sicherer Handhabung eine zuverlässige Entfernung der Gegenstände (7) auch bei beengten Platzverhältnissen gewährleistet, wird erfindungsgemäß vorgeschlagen, dass die Handhabe (3) als aus mindestens zwei Griffteilen (3a, 3b) bestehend so ausgebildet ist, dass die Handhabe (3) aus mindestens zwei gegenüber der Instrumentenlängsachse (1a) abgewinkelt ausgebildeten Griffteilen (3a, 3b) besteht, wobei mindestens ein Griffteil (3b) gegenüber dem mindestens einen anderen Griffteil (3a) verschwenkbar ist.

## Beschreibung

Die Erfindung betrifft ein medizinisches Instrument zum Entfernen von Gegenständen aus engen Kanälen, mit einem Schaft und einem im Schaft gelagerten Betätigungselement, an dessen distalem Ende eine hakenförmig Abwinklung ausbildbar ist, wobei das distale Ende des Betätigungselements über eine Handhabe zwischen einer im Wesentlichen gestreckten Form und einer die hakenförmige Abwinklung aufweisenden Form verstellbar ist.

Derartige hakenförmige medizinische Instrumente sind beispielsweise aus der Hals-Nasen-Ohren-Heilkunde bekannt, um mit ihnen beispielsweise Zerumen, das sogenannte Ohrenschmalz, oder andere Fremdkörper aus dem äußeren Gehörgang zu entfernen. Nachteilig bei den bekannten Instrumenten ist, dass es schwierig ist, das hakenförmige distale Ende des Schaftes hinter dem zu entfernenden Gegenstand platziert zu bekommen, um den Gegenstand anschließend mittels des Hakens aus dem Gehörgang herauszuziehen. Je nach den vorliegenden Platzverhältnissen besteht in der Praxis immer die Gefahr, dass beim Einführen des hakenförmigen Schaftes der zu entfernende Gegenstand weiter in den Gehörgang hinein gedrückt wird.

Ein derartiges medizinisches Instrument ist beispielsweise aus der DE 195 14 534 C2 bekannt. Bei diesem bekannten medizinischen Instrument ist die Handhabe als stabförmig Verlängerung des Schaftes ausgebildet. Diese im Querschnitt runde stabförmige Handhabe ermöglicht zwar den Bau eines schlanken und platzsparenden Instruments, jedoch gewährleistet die stabförmige Ausbildung der Handhabe keine jederzeit sichere und lagefeste Positionierung in der Hand des Bedieners.

Davon ausgehend liegt der Erfindung die **Aufgabe** zugrunde, ein medizinisches Instrument zum Entfernen von Gegenständen aus engen Kanälen zu schaffen, das bei einfacher und sicherer Handhabung eine zuverlässige Entfernung der Gegenstände auch bei beengten Platzverhältnissen gewährleistet.

Die **Lösung** dieser Aufgabenstellung ist erfindungsgemäß dadurch gekennzeichnet, dass die Handhabe als aus mindestens zwei Griffteilen bestehend so ausgebildet ist, dass die Handhabe aus mindestens zwei gegenüber der Instrumentenlängsachse abgewinkelt ausgebildeten Griffteilen besteht, wobei mindestens ein Griffteil gegenüber dem mindestens einen anderen Griffteil verschwenkbar ist.

Durch die erfindungsgemäße Ausgestaltung der Handhabe als scherenartig ausgebildeter und gegenüber der Instrumentenlängsachse abgewinkelter Griff mit mindestens zwei Griffteilen liegt das erfindungsgemäß ausgebildete medizinische Instrument sicher und griffig in der Hand des Benutzers und ermöglicht so eine einfache, zielgenaue und sichere Handhabung des Instruments.

Gemäß einer praktischen Ausführungsform der Erfindung wird vorgeschlagen, dass die Handhabe ein mit dem Schaft verbundenes starres Griffteil sowie ein gegenüber dem starren Griffteil verschwenkbares Griffteil aufweist, wobei das verschwenkbare Griffteil mit dem Betätigungselement in Wirkverbindung steht. Die Verwendung des mit dem Betätigungselement in Wirkverbindung stehenden verschwenkbaren Griffteils ermöglicht eine dosierte axiale Verschiebung des Betätigungselements und somit eine sichere und schonende Behandlung des Patienten.

Das Überführen des distalen Endes des Betätigungselements in die hakenförmig abgewinkelte Form erfolgt erfindungsgemäß vorteilhafterweise selbsttätig, was durch die Verwendung eines aus einem vorgebogenen Material bestehenden Betätigungselements ermöglicht wird.

Als Material zur erfindungsgemäßen Ausbildung zumindest des distalen Endes des Betätigungselements sind insbesondere sogenannte superelastische Werkstoffe, wie beispielsweise NiTi-Legierungen, geeignet, die die notwendige Elastizität und Steifigkeit besitzen, um einerseits in eine im Wesentlichen gestreckte Form gebogen zu werden und andererseits Dank des Memory-Effektes wieder die vorgebogene Hakenform einnehmen, wobei sie in der abgewinkelten Form ausreichend stabil sind, um den zu entfernenden Gegenstand auch entgegen einem gewissen Widerstand aus dem Kanal herausziehen zu können.

Zur Ausbildung des distalen Endes des Betätigungselements wird gemäß einer ersten Ausgestaltungsform der Erfindung vorgeschlagen, dass das distale Ende des Betätigungselements als geschlossene Drahtschlinge ausgebildet ist.

Mit einer zweiten Ausführungsform der Erfindung wird vorgeschlagen, dass das distale Ende des Betätigungselements als streifenförmiges flächiges Blech ausgebildet ist.

Weiterhin wird mit der Erfindung vorgeschlagen, dass das freie distale Ende des Betätigungselements stumpf ausgebildet ist, um auszuschließen, dass durch das zunächst in gestreckter Form in den Kanal eingeführte freie Ende des Betätigungselements eine Verletzung des zu untersuchenden Kanals hervorgerufen werden kann.

Schließlich wird mit der Erfindung vorgeschlagen, dass der Schaft über ein Spekulum in den zu untersuchenden Kanal einführbar ist. Diese trichterförmigen Instrumente sind beispielsweise aus dem HNO-Bereich bekannt, um in Körperöffnungen, wie beispielsweise den äußeren Gehörgang, eingesetzt zu werden. Das Spekulum verhindert, dass der zu untersuchende Kanal beim Einführen von Untersuchungsinstrumenten verletzt wird.

Weitere Merkmale und Vorteile der Erfindung ergeben sich anhand der zugehörigen Zeichnung, in der zwei Ausführungsbeispiele eines erfindungsgemäßen medizinischen Instruments zum Entfernen von Gegenständen aus engen Kanälen nur beispielhaft dargestellt sind, ohne die Erfindung auf diese dargestellten Ausführungsbeispiele zu beschränken. In der Zeichnung zeigt:
- Fig. 1: eine schematische Seitenansicht eines erfindungsgemäßen medizinischen Instruments mit eingezogenem Schaft;
- Fig. 2: eine perspektivische Ansicht des Details II gemäß Fig. 1, eine erste Ausführungsform des distalen Endes des Betätigungselements darstellend;
- Fig. 3: eine perspektivische Ansicht des Details II gemäß Fig. 1, eine zweite Ausführungsform des distalen Endes des Betätigungselements darstellend;
- Fig. 4a: eine schematische Darstellung der Verwendung eines erfindungsgemäßen medizinischen Instruments während des Einführens;
- Fig. 4b: eine Darstellung gemäß Fig. 4a, jedoch die Arbeitsstellung darstellend und
- Fig. 5: eine schematische Seitenansicht eines Spekulums.

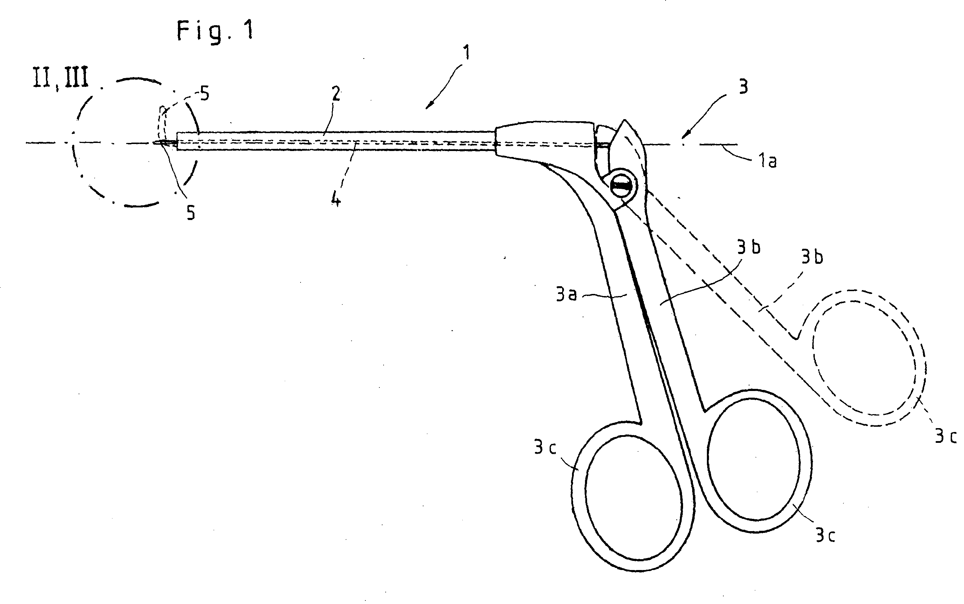

Das in Fig. 1 nur beispielhaft schematisch dargestellte medizinische Instrument 1 zum Entfernen von Gegenständen aus engen Kanälen besteht im Wesentlichen aus einem Schaft 2, an dessen proximalem Ende eine Handhabe 3 angeordnet ist, die aus einem mit dem Schaft 2 verbundenen starren Griffteil 3a sowie einem gegenüber dem starren Griffteil 3a verschwenkbaren Griffteil 3b besteht. Um das Halten und Bedienen der Handhabe 3 zu erleichtern, weisen beide Griffteile 3a und 3b darüber hinaus Fingerringe 3c auf.

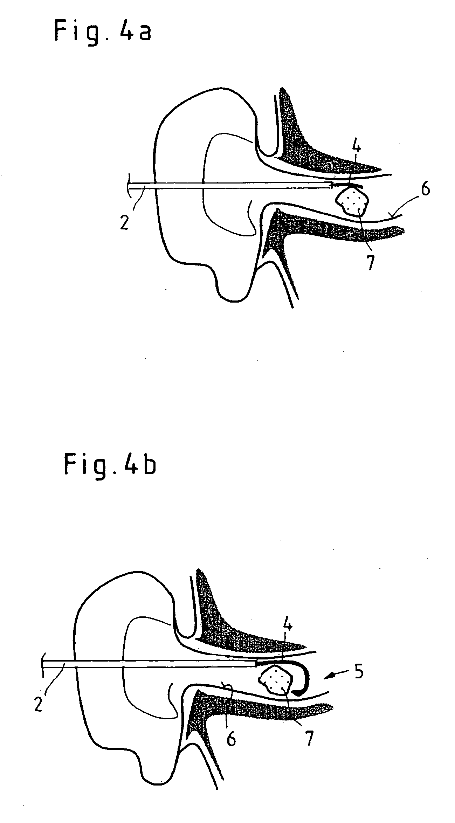

Innerhalb des Schaftes 2 ist ein mit dem verschwenkbaren Griffteil 3b der Handhabe 3 in Wirkverbindung stehendes Betätigungselement 4 gelagert, dessen distales Ende eine Abwinklung 5 bildend aus dem distalen Ende des Schaftes 2 herausschiebbar ist, um einen in einem Kanal 6 angeordneten Fremdkörpers 7 hintergreifen zu können, wie dies insbesondere in Fig. 4b dargestellt ist.

Wie aus Fig. 1 ersichtlich, stehen die am distalen Ende des Betätigungselements 4 ausgebildete Abwinklung 5 und der verschwenkbare Griffteil 3b der Handhabe 3 über das im Schaft 2 gelagerte Betätigungselement 4 derart in Wirkverbindung miteinander, dass durch das Verstellen des Griffteils 3b der Handhabe 3 das distale Ende des Betätigungselements 4 aus der zumindest teilweise im Schaft gelagerten gestreckten Grundstellung (durchgezogene Darstellung gemäß Fig. 1) in die aus dem Schaft 2 ausgefahrene, die Abwinklung 5 ausbildende Arbeitsstellung (gestrichelte Darstellung gemäß Fig. 1) bzw. umgekehrt überführbar ist. Die jeweils zugehörige Stellung des verschwenkbaren Griffteils 3b der Handhabe 3 ist in der Abbildung Fig. 1 ebenfalls durchgezogen (für die gestreckte Grundstellung) und gestrichelt (für die abgewinkelte Arbeitsstellung) dargestellt.

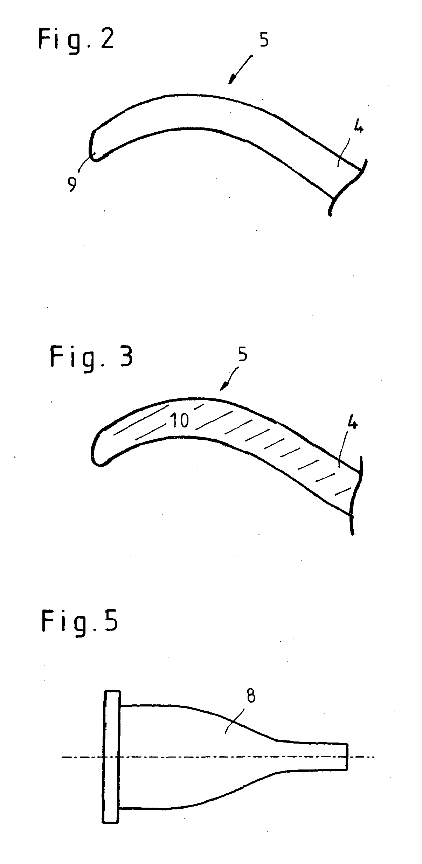

Um Verletzungen des Kanals 6, beispielsweise dem äußeren Gehörgang eines Ohres, zu vermeiden und das Einführen des Schaftes 2 in den Gehörgang 3 zu erleichtern, wird in der Praxis zunächst ein trichterförmiges Spekulum 8 in den Gehörgang 6 eingesetzt. Das in Abbildung Fig. 5 dargestellte Spekulum 8 wurde in den Abbildungen Fig. 4a und 4b aus Gründen der besseren Übersichtlichkeit weggelassen.

Zur Ausbildung der hakenförmigen Abwinklung 5 am distalen Ende des Betätigungselements 4 besteht zumindest das distale Ende des Betätigungselements 4 aus einem elastischen Material, wie beispielsweise einer superelastischen NiTi-Legierung, das in eine hakenförmige Form vorgebogen ist. Die Elastizität des Materials erlaubt es jedoch, den Schaft in eine im Wesentlichen gestreckte Form zu überführen, um ihn mittels des Schaftes 2 in den zu untersuchenden Kanal 6 einführen zu können. Sobald das distale Ende des Betätigungselements 4 den Schaft 2 wieder verlässt, nimmt es selbsttätig wieder die vorgebogene hakenförmige Form ein.

In den Abbildungen 2 und 3 sind zwei Ausführungsbeispiele zur Ausgestaltung des die Abwinklung 5 bildenden distalen Endes des Betätigungselements 4 schematisch dargestellt.

Bei der in Fig. 2 dargestellten ersten Ausführungsform ist das die Abwinklung 5 ausbildende distale Ende des Betätigungselements 4 als geschlossene Drahtschlinge 9 ausgebildet. Dahingegen zeigt die in Fig. 3 dargestellte zweite Ausführungsform, die Ausbildung des die Abwinklung 5 ausbildenden distalen Endes des Betätigungselements 4 als streifenförmiges flächiges Blech 10.

Wie weiterhin aus Fig.1, 2 und 3 ersichtlich, ist es möglich die Abwinklung 5 so auszubilden, dass diese sich von der Instrumentenlängsachse 1a fort nach oben oder nach unten abwinkelt. Selbstverständlich ist es auch möglich eine seitliche Auslenkung der Abwinklung 5 auszugestalten.

Die Handhabung des zuvor beschriebenen medizinischen Instruments 1 zum Entfernen von Gegenständen 7 aus engen Kanälen 6 geschieht wie folgt:

Die Abbildungen Fig. 4a und 4b zeigen schematisch die praktische Anwendung des zuvor beschriebenen medizinischen Instruments am Beispiel eines aus dem äußeren Gehörgang 6 eines Ohres zu entfernenden Fremdkörpers 7, bei dem es sich beispielsweise um verhärtetes Zerumen, das sogenannte Ohrenschmalz, handeln kann.

Das in Abbildung Fig. 5 dargestellte Spekulum 8 wurde in den Abbildungen Fig. 4a und 4b aus Gründen der besseren Übersichtlichkeit weggelassen. Ebenso wurden weitere medizinische Instrumente, wie beispielsweise ein Otoskop zur visuellen Überwachung der Behandlung, nicht dargestellt.

Über das Spekulum 8 wird der Schaft 2 des medizinischen Instruments 1 in den Gehörgang 6 eingeführt, bis das distale Ende des Schaftes 2 fast den zu entfernenden Fremdkörper 7 erreicht hat. Anschließend verschwenkt der Bediener des medizinischen Instruments 1den verschwenkbaren Griffteil 3b der Handhabe 3 in die in Fig. 1 gestrichelt dargestellte Position, wodurch das distale Ende des Betätigungselements 4 aus dem distalen Ende des Schaftes 2 herausgeschoben wird, wie dies in Fig. 4a dargestellt ist.

Das aus dem Schaft 2 austretende distale Ende des Betätigungselements 4 passiert den zu entfernenden Fremdkörper 7 und bildet bei weiterem Herausschieben des Betätigungselements 4 aus dem Schaft 2 aufgrund der Vorspannung und des Memory-Effekts des Materials hinter dem Fremdkörper 7 selbsttätig die vorgeformte Abwinklung 5 aus, wie diese der Abbildung Fig. 4b zu entnehmen ist.

Zum Entfernen des Fremdkörpers 7 aus dem Gehörgang 6 muss der Bediener des medizinischen Instruments 1 jetzt nur noch bei weiterhin verschwenktem Griffteil 3b den Schaft 2 nach außen aus dem Gehörgang 6 herausziehen. Die den Fremdkörper 7 hintergreifende Abwinklung 5 nimmt diesen mit und entfernt ihn aus dem Gehörgang 6.

Neben der Auswahl eines geeignet elastischen Materials zur Ausbildung des distalen Endes des Betätigungselements 4, um ein wiederholtes Strecken und Zurückbiegen des distalen Endes des Betätigungselements 4 zu ermöglichen, ist das Material hinsichtlich der Materialsteifigkeit und/oder Materialstärke so auszuwählen, dass die den zu entfernenden Fremdkörper 7 hintergreifende Abwinklung 5 sich nicht sofort zurück biegt, wenn durch den Fremdkörper 7 eine Widerstandskraft auf die hakenförmige Abwinklung 5 ausgeübt wird.

Ein solchermaßen ausgebildetes medizinisches Instrument zum Entfernen von Gegenständen 7 aus engen Kanälen 6 zeichnet sich dadurch aus, dass es bei einfachem Aufbau und einfacher Handhabung ein zuverlässige Entfernung der Fremdkörper 7 aus dem zu untersuchenden Kanal 6 gewährleistet. Durch die Ausgestaltung der Handhabe 3 als scherenartig ausgebildeter Griff mit mindestens zwei Griffteilen 3a, 3b liegt das solchermaßen ausgebildete medizinische Instrument 1 sicher und griffig in der Hand des Benutzers und ermöglicht so eine einfache, zielgenaue und sichere Handhabung des medizinischen Instruments 1.

### Bezugiszeichenliste

- 1: medizinisches Instrument
- 1a: Instrumentenlängsachse
- 2: Schaft
- 3: Handhabe
- 3a: starres Griffteil
- 3b: verschwenkbares Griffteil
- 3c: Fingerring
- 4: Betätigungselement
- 5: Abwinklung
- 6: Kanal/Gehörgang
- 7: Fremdkörper
- 8: Spekulum
- 9: Drahtschlinge
- 10: flächiges Blech

## Patentansprüche

1. Medizinisches Instrument zum Entfernen von Gegenständen aus engen Kanälen, mit einem Schaft (2) und einem im Schaft (2) gelagerten Betätigungselement (4), an dessen distalem Ende eine hakenförmig Abwinklung (5) ausbildbar ist, wobei das distale Ende des Betätigungselements (4) über eine Handhabe (3) zwischen einer im Wesentlichen gestreckten Form und einer die hakenförmige Abwinklung (5) aufweisenden Form verstellbar ist,
**dadurch gekennzeichnet,**
**dass** die Handhabe (3) aus mindestens zwei gegenüber der Instrumentenlängsachse (1a) abgewinkelt ausgebildeten Griffteilen (3a, 3b) besteht, wobei mindestens ein Griffteil (3b) gegenüber dem mindestens einen anderen Griffteil (3a) verschwenkbar ist.

2. Medizinisches Instrument nach Anspruch 1, **dadurch gekennzeichnet, dass** die Handhabe (3) ein mit dem Schaft (2) verbundenes starres Griffteil (3a) sowie ein gegenüber dem starren Griffteil (3a) verschwenkbares Griffteil (3b) aufweist, wobei das verschwenkbare Griffteil (3b) mit dem Betätigungselement (4) in Wirkverbindung steht.

3. Medizinisches Instrument nach Anspruch 1 oder 2, **dadurch gekennzeichnet, dass** das distale Ende des Betätigungselements (4) selbsttätig die die hakenförmige Abwinklung (5) aufweisende Form einnimmt.

4. Medizinisches Instrument nach einem der Ansprüche 1 bis 3, **dadurch gekennzeichnet, dass** das distale Ende des Betätigungselements (4) aus einem vorgebogenen elastischen Material besteht.

5. Medizinisches Instrument nach Anspruch 4, **dadurch gekennzeichnet, dass** das distale Ende des Betätigungselements (4) aus einer NiTi-Legierung, besteht.

6. Medizinisches Instrument nach einem der Ansprüche 1 bis 5, **dadurch gekennzeichnet, dass** das distale Ende des Betätigungselements (4) als geschlossene Drahtschlinge (9) ausgebildet ist.

7. Medizinisches Instrument nach einem der Ansprüche 1 bis 5, **dadurch gekennzeichnet, dass** das distale Ende des Betätigungselements (4) streifenförmig ausgebildet ist.

8. Medizinisches Instrument nach einem der Ansprüche 1 bis 7, **dadurch gekennzeichnet, dass** das freie distale Ende des Betätigungselements (4) stumpf ausgebildet ist.

9. Medizinisches Instrument nach einem der Ansprüche 1 bis 8, **dadurch gekennzeichnet, dass** der Schaft (2) über ein Spekulum (8) in den Kanal (6) einführbar ist.
